# EUROPEAN PATENT APPLICATION

(11) **EP 3 978 912 A1**
(43) Date of publication of application: **06.04.2022**
(21) Application number: 20199878.8
(22) Date of filing: 02.10.2020
(51) Int. Cl.: G01N 27/414, G01N 33/487

(54) **APPARATUS, METHODS AND COMPUTER PROGRAMS FOR ANALYSING CELLULAR SAMPLES**

(71) Applicant: Nokia Technologies Oy, 02610 Espoo (FI)
(72) Inventor: ZHENG, Mingde, New Providence, NJ New Jersey 07974 (US)
(74) Representative: Swindell & Pearson Limited

(57) **Abstract**

Systems and methods for analysing cellular samples. The systems comprises an apparatus comprising means for providing a stimulating signal to a cellular sample wherein the stimulating signal stimulates the cellular sample so as to modify one or more electrical properties of the cellular sample and providing a plurality of electrical input signals having a plurality of different frequencies to the cellular sample. The apparatus also comprises means for receiving electrical output signals from the cellular sample where the electrical output signals correspond to the electrical input signals and the values of the electrical output signals are determined by the modified electrical properties of the cellular sample; and enabling spectroscopic analysis of the electrical output signals to identify a type of cell in the cellular sample from the modified electrical properties.

## Description

### TECHNOLOGICAL FIELD

Examples of the disclosure relate to apparatus, methods and computer programs for analysing cellular samples. Some relate to apparatus, methods and computer programs for analysing cellular samples in-vivo.

### BACKGROUND

Analysis of cellular samples can provide useful information about a subject. This can be used for diagnosis, health monitoring or any other suitable purpose.

### BRIEF SUMMARY

According to various, but not necessarily all, examples of the disclosure there is provided an apparatus comprising means for: providing a stimulating signal to a cellular sample wherein the stimulating signal stimulates the cellular sample so as to modify one or more electrical properties of the cellular sample; providing a plurality of electrical input signals having a plurality of different frequencies to the cellular sample; receiving electrical output signals from the cellular sample where the electrical output signals correspond to the electrical input signals and the values of the electrical output signals are based on the modified one or more electrical properties of the cellular sample; and enabling spectroscopic analysis of the electrical output signals to identify a type of cell in the cellular sample from the modified one or more electrical properties.

The analysis may comprise comparing the received output electrical signals with a database of known electrical output signal values of cellular samples so as to identify at least one cell type within the cellular sample.

The database of known electrical output signal values may be obtained from in-vitro measurements of known cellular samples.

The analysis may comprise impedance spectroscopy of the electrical output signals.

The plurality of electrical input signals may comprise a carrier waveform where the carrier waveform is frequency locked to a preselected frequency.

The preselected frequency may correspond to a component of the cellular sample.

The plurality of electrical input signals may have a frequency that is tuned to an accuracy of millihertz.

The plurality of electrical input signals may comprise a phase locked signal.

The means may be for providing the electrical input signals simultaneously with the stimulating signal.

The means may be for providing a plurality of different stimulating signals at different times to enable a plurality of different modified one or more electrical properties of the cellular sample to be analysed using the spectroscopic analysis.

The different modified one or more electrical properties may provide different identifying characteristics of cell types in the cellular sample.

The one or more electrical properties that are modified by the stimulating signal may comprise one or more of resistance, capacitance, impedance, dielectrics, phase.

The stimulating signal may comprise one or more of electrical signals, mechanical signals, acoustic signals, photonic signals, magnetic signals, electromagnetic signals.

At least one of the stimulating signal or the plurality of electrical input signals may be provided in programmed waveforms.

The stimulating signal may cause a temporary modification of the one or more electrical properties of the cellular sample.

The cellular sample may comprise an in-vivo sample.

According to various, but not necessarily all, examples of the disclosure there is provided a method comprising: providing a stimulating signal to a cellular sample wherein the stimulating signal stimulates the cellular sample so as to modify one or more electrical properties of the cellular sample; providing a plurality of electrical input signals having a plurality of different frequencies to the cellular sample; receiving electrical output signals from the cellular sample where the electrical output signals correspond to the electrical input signals and the values of the electrical output signals are based on the modified one or more electrical properties of the cellular sample; and enabling spectroscopic analysis of the electrical output signals to identify a type of cell in the cellular sample from the modified one or more electrical properties.

According to various, but not necessarily all, examples of the disclosure there is provided a computer program comprising computer program instructions that, when executed by processing circuitry, cause: providing a stimulating signal to a cellular sample wherein the stimulating signal stimulates the cellular sample so as to modify one or more electrical properties of the cellular sample; providing a plurality of electrical input signals having a plurality of different frequencies to the cellular sample; receiving electrical output signals from the cellular sample where the electrical output signals correspond to the electrical input signals and the values of the electrical output signals are determined by the modified one or more electrical properties of the cellular sample; and enabling spectroscopic analysis of the electrical output signals to identify a type of cell in the cellular sample from the modified one or more electrical properties.

### BRIEF DESCRIPTION

Some examples will now be described with reference to the accompanying drawings in which:
- FIG. 1: shows an example of the subject matter described herein;
- FIG. 2: shows another example of the subject matter described herein;
- FIG. 3: shows another example of the subject matter described herein;
- FIGS. 4A and 4B: show another example of the subject matter described herein;
- FIGS. 5A and 5B: show another example of the subject matter described herein;
- FIGS. 6: shows another example of the subject matter described herein;
- FIG. 7: shows another example of the subject matter described herein;
- FIG. 8: shows another example of subject matter described herein;
- FIG. 9: shows another example of the subject matter described herein;
- FIGS. 10A to 10C: show another example of the subject matter disclosed herein; and
- FIG. 11: shows another example of the subject matter disclosed herein.

### DETAILED DESCRIPTION

Examples of the disclosure relate to systems and methods for analysing cellular samples. In examples of the disclosure a stimulating signal is provided to the cellular sample to stimulate an electrical response that can be analysed by applying electrical input signals to the cellular sample.

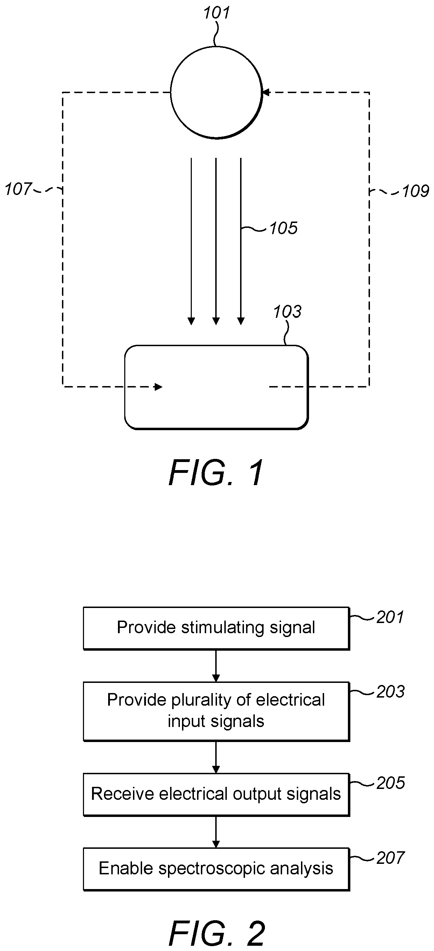

Fig. 1 schematically shows an example apparatus 101 for analysing a cellular sample 103. The cellular sample 103 can comprise an in-vivo sample. The cellular sample 103 can comprise any biological cellular matter. The cellular sample 103 can comprise a single type of cell or can comprise a plurality of different types of cells.

The apparatus 101 comprises means for providing a stimulating signal 105 to the cellular sample 103. The stimulating signal 105 can comprise any signal that can stimulate the cellular sample 103 so as to modify one or more electrical properties of the cellular sample 103. The stimulating signal 105 can be configured so that it does not cause irreversible damage to the cells within the cellular sample 103 or does not kill the cells within the cellular sample 103.

The stimulating signal 105 can causes a temporary modification of the electrical properties of the cellular sample 103. In such examples the stimulating signal 105 could be applied to the cellular sample 103 for a period of time and the electrical properties of the sample could revert to their original state once the stimulating signal 105 is stopped.

The electrical properties of the cellular sample 103 that are modified can be any properties that can be measured or detected by using a plurality of input electrical signals. The electrical properties that are modified could comprise any one or more of resistance, capacitance, impedance, dielectric, phase or any other suitable property. The electrical properties that are modified could comprise properties of an entire cell or properties of just part of a cell.

The stimulating signal 105 could comprise any suitable type of signal, for instance it could comprise any one or more of electrical signals, mechanical signals, acoustic signals, photonic signals, magnetic signals, electromagnetic signals or any other suitable type of signal. The stimulating signal 105 could cause any change within the cells of the cellular sample 103 that results in the modification of the electrical properties of the cells. In some examples the stimulating signal 105 could cause mechanical or structural changes to the cells which result in the modification to the electrical properties. For example, a change in shape of the cells or parts of the cells could provide a measurable change in electrical properties. In other examples the stimulating signal could cause a chemical or electrical change or any other suitable type of change within the structure of the cells of the cellular sample 103.

The apparatus 101 can be configured to provide the stimulating signal 105 in programmed waveforms. The timing, intensity, frequency and any other suitable property of the stimulating signal 105 can be programmed through the apparatus 101.

The apparatus 101 can comprise means that enables the stimulating signal 105 to be provided to the cellular sample 103. The type of means that are used will depend on the type of stimulating signal 105 that is used. For instance, if the stimulating signal 105 comprises an electrical signal then one or more electrodes can be applied to the cellular sample 103 to enable the electrical signal to be provided to the cellular sample 103. Similarly, if the stimulating signal 105 comprises an acoustic signal then the means for providing the stimulating signal 105 to the cellular sample 103 could be an acoustic transducer or any other suitable means.

In some examples a plurality of different stimulating signals 105 can be applied to the cellular sample 103. The different stimulating signals 105 can be applied to the cellular sample 103 at different times. The different stimulating signals can enable different modifications of the electrical properties of the cellular sample 103 to be analysed. The different stimulating signals 105 could be different types of stimulating signals 105 or could be stimulating signals 105 of the same type but having different characteristics. For instance, they could be electrical input signals having different frequencies.

The apparatus 101 also comprises means for providing a plurality of electrical input signals 107 having a plurality of different frequencies to the cellular sample 103. The plurality of electrical input signals 107 can comprise an alternating electrical current. The different electrical input signals 107 can have different waveforms so that the waveforms of different electrical input signals can have different amplitudes, shapes, frequencies, cycles or any other suitable parameter.

The electrical input signals 107 can comprise signals that enable the modification of the electrical properties of the cellular sample 103 that is caused by the stimulating signal 105 to be measured or detected. The electrical input signals 107 are provided at a plurality of different frequencies so that the modified electrical properties of the cellular sample 103 can be measured at different frequencies.

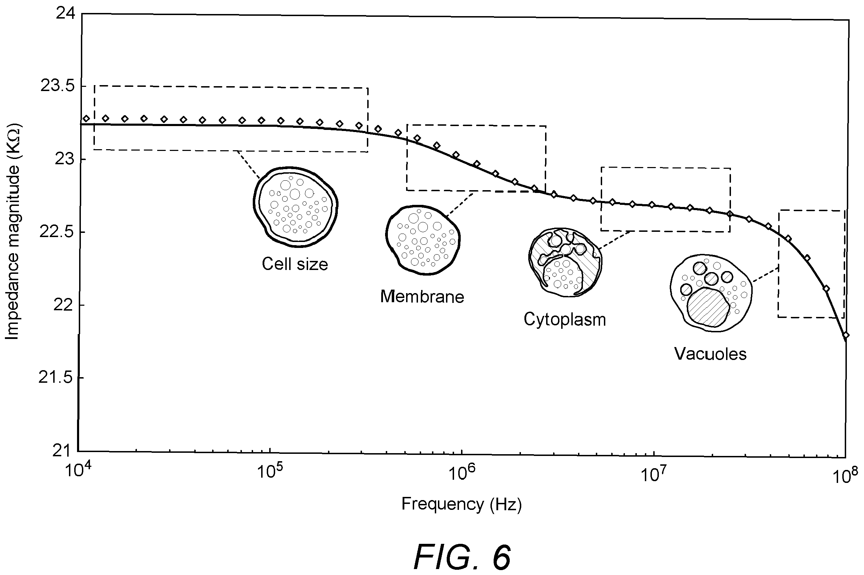

The plurality of electrical input signals 107 comprise a carrier waveform where the carrier waveform is frequency locked to a preselected frequency. The preselected frequency can correspond to a component of the cellular sample 103. For example, the preselected frequency can be tuned to correspond to a particular part of the cells that are expected to be within the cellular sample 103. For instance the preselected frequency could be tuned to measure a response in the extracellular matrix, cell membrane, cytosol, organelles or any other suitable part of the cells within the cellular sample 103. As shown in Fig. 6 the different parts of a cell are known to have different electrical responses at different frequencies and so by tuning the frequency of the electrical input signal 107 different parts of the cells can be analysed.

The pre-selected frequency can be tuned to a high accuracy. In some examples the plurality of electrical input signals have a frequency that is tuned to an accuracy of millihertz. In some examples the electrical input signal 107 can comprise one or more phase locked signals. This can enable small modifications of the electrical properties of the cells to be detected by the electrical input signals 107.

The apparatus 101 can be configured so that the electrical input signal 107 can be applied simultaneously to the stimulating signals 105 so that the apparatus 101 can provide the two different types of signal at the same time. This can ensure that the electrical input signal 107 is applied while the electrical properties of the cells are modified by the stimulating signals 105.

The means for providing the plurality of electrical input signals 107 could comprise one or more electrodes that can be applied to the cellular sample 103 or any other suitable means. The one or more electrodes could provide a path for current flow from the electrodes into the cellular sample 103.

The apparatus 101 also comprises means for receiving electrical output signals 109 from the cellular sample 103. The electrical output signals 109 correspond to the electrical input signals 107 in that they can be provided by the electrical input signals 107 passing through the cellular sample 103 so that the values of one or more properties of the electrical output signals 109 are determined by the modified electrical properties of the cellular sample 103, where the modification of the electrical properties of the cellular samples 103 is caused by the stimulating signal 105. This enables the electrical output signals 109 to provide an indication of the modified electrical properties of the cells within the cellular sample 103.

The apparatus 101 also comprises means for enabling spectroscopic analysis of the electrical output signals 109 to identify a type of cells in the cellular sample 103 from the modified electrical properties. In some examples the apparatus 101 can be configured to process the received electrical output signals 109 to perform the spectroscopic analysis. In other example the apparatus 101 could be configured to transmit the received electrical input signals 109 to another apparatus or device to enable the another apparatus or device to perform the spectroscopic analysis.

The spectroscopic analysis can comprise comparing the received output electrical signals 109 with a database of known electrical output signal values of known cellular samples 103 so as to identify at least one cell type within the cellular sample 103. The database of known electrical output signal values can be obtained from in-vitro measurements of known cellular samples 103. The database can be stored in the apparatus 101 or could be stored in another device that can be accessed by the apparatus 101 when the spectroscopic analysis is being performed.

The type of spectroscopic analysis that is used will depend upon the electrical properties of the cellular sample 103 that are modified by the stimulating signal 105. For instance, if the stimulating signal 105 modifies the impedance of the cellular sample 103 then the spectroscopic analysis would comprise impedance spectroscopy of the electrical output signals. The spectroscopic analysis of the electrical output signals 109 can comprises electrochemical impedance spectroscopy (EIS), electrical cell-substrate impedance spectroscopy (ECIS), bio-electrical impedance vector analysis (BIVA), bio-electrical impedance analysis (BIA), electrical impedance spectroscopy (EIS), bio-electrical impedance spectroscopy (BIS) or any other suitable type of spectroscopy.

The spectroscopic analysis can comprise analysing the output signal 109 for a plurality of different frequencies input signal 107 and comparing these with expected responses for known types of cells. The expected responses for the known cell types can be obtained from measurements of known types of cells and stored in a database that can be accessed by the apparatus 101 that is performing the analysis. If the output signals 109 from a cellular sample are shown to have a good correlation with, or match, an expected response for a type of cell then this indicates that the cells within the cellular sample 103 comprise that type of cell.

Fig. 2 shows a method that can be implemented using an apparatus 101 as shown in Fig. 1.

The method comprises, at block 201, providing a stimulating signal 105 to a cellular sample 103. The stimulating signal 105 is configured to cause stimulation of cells within the cellular sample 103 so as to modify one or more electrical properties of the cellular sample 103. The stimulating signal 105 can be any type of signal that causes a modification in one or more electrical properties of cells within the cellular sample 103. The stimulating signal 105 could comprise any one or more electrical signals, mechanical signals, acoustic signals, photonic signals, magnetic signals, electromagnetic signals including any other suitable type of signal.

At block 203 the method comprises providing a plurality of electrical input signals 107 having a plurality of different frequencies to the cellular sample 103. In some examples the plurality of electrical input signals 107 are applied to the cellular sample 103 simultaneously to the stimulating signal 105. This enables the electrical input signals 107 to be used to detect the modification of the electrical properties of the cellular sample 103 caused by the stimulating signal 105.

The plurality of electrical input signals 107 can be provided at a plurality of different frequencies. The different frequencies can be selected so as to enable the response of different parts of the cells within the cellular sample 103 to be analysed. The different frequencies can be selected to correspond to the known frequency response of different parts of the cells as shown in Fig. 6 for example.

The different electrical input signals 107 having different frequencies can be provided at different times. This can enable a first frequency electrical input signal 107 to be used at a first time and a second, different frequency electrical input signal 107 to be used at a second time. This can enable the different frequency responses of the different parts of the cells to be analysed.

At block 205 the method comprises receiving electrical output signals 109 from the cellular sample 103. The electrical output signals 109 correspond to the electrical input signals 107 in that they are provided by the electrical input signals 107 passing through the cellular sample 103. As the received electrical output signals 109 have passed through the cellular sample 103 the values of the electrical output signals 109 are determined by the modified electrical properties of the cellular sample 103.

At block 207 the method comprises enabling 207 spectroscopic analysis of the electrical output signals 109 to identify a type of cell in the cellular sample 103 from the modified electrical properties. The spectroscopic analysis comprises comparing the received output electrical signals 109 for a range of frequencies with a database of known electrical output signal values of cellular samples so as to identify at least one cell type within the cellular sample 103.

The use of the stimulating signal 105 to modify the electrical properties of the cellular sample provides a unique expected output signal 109 for a given type of cell for a range of frequencies of the electrical input signal 107, where the received output signals 109 corresponding to a range of frequencies of the electrical input signals 107 can then be used to identify types of cells within a cellular sample 103.

In some examples the method of Fig. 2 can be repeated for different stimulating signal 105 so as to obtain further output signal 109 that can provide for improved accuracy of the identification of the types of cells.

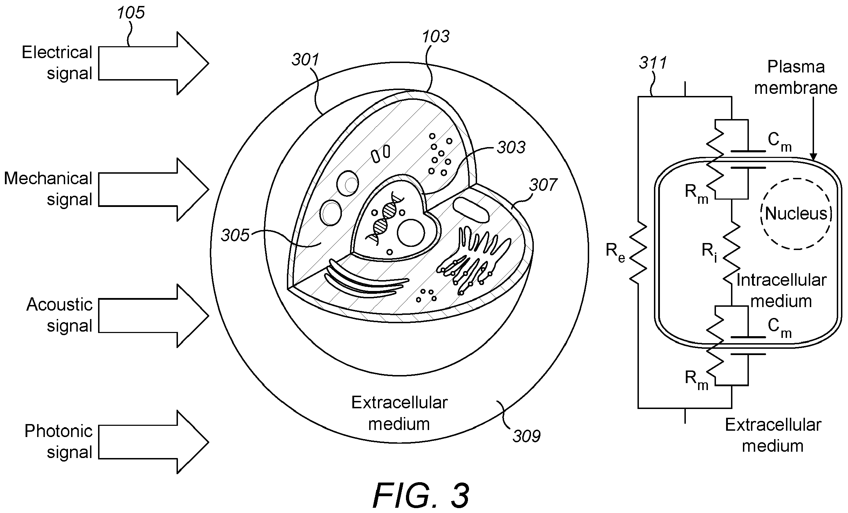

Fig. 3 schematically shows different types of stimulating signals 105 that can be applied to a cellular sample 103. It is to be appreciated that in examples of the disclosure a single type of stimulating signal 105 could be provided by the apparatus 101 and the different types are shown here for illustrative purposes. In other examples the apparatus 101 could be configured to provide different types of stimulating signal 105 to enable different modifications to the electrical properties of the cellular sample 103 to be triggered.

The first type of stimulating signal 105 signal comprises an electrical signal. The electrical signal can comprise a pulse train comprising a series of discrete pulses provided at a predetermined frequency and amplitude. Other types of electrical input signals could be provided in other examples of the disclosure.

The second type of stimulating signal 105 comprises a mechanical signal. The mechanic signal could comprise any signal that enables a mechanical force to be applied to the cells within the cellular sample 103. The mechanical force can enable a stress and/or strain to be applied to the cells within the cellular sample 103 which can cause a change in shape of the cells. This change in shape can result in modification of the electrical properties of the cells. The mechanical signal could also be provided as a pulse train comprising a series of discrete pulses provided at a predetermined frequency and amplitude or as any form of signal.

A third type of stimulating signal 105 could comprise an acoustic signal. The acoustic signal could comprise an ultrasonic signal such as a sonication signal or any other suitable type of signal. The acoustic signal could cause vibrations of the cells or parts of the cells that can modify electrical properties of the cells. The frequencies of the acoustic signals could be selected to correspond to one or more resonant frequencies of components of the cells.

A fourth type of stimulating signal 105 could comprise a photonic signal. The photonic signal could comprise an electromagnetic signal that causes modification of the electrical properties of the cells within the cellular sample 103. The photonic signal could be provided by light emitting diodes or by any other suitable source.

It is to be appreciated that other types of stimulating signal 105 could be used in other examples of the disclosure that are not shown. For instance, in some examples a magnetic signal could be used. The magnetic signal could be provided by one or more electromagnets positioned close to the cellular sample 103 or by any other suitable means. In some examples the stimulating signal 105 could comprise electromagnetic signals such as millimetre-wave signals. The millimetre-wave signals could comprise high frequency radio frequency signals that are directed towards the cellular sample 103. The millimetre-wave signals could be provided by one or more transmitters configured to direct a signal towards the cellular sample 103.

Each of the different types of stimulating signal 105 are configured so that they are provided at sub-lethal values to the cellular sample 103. That is, the stimulating signals 105 do not cause irreversible damage to the cells within the cellular sample 103.

The stimulating signal 105 is configured to be provided temporarily to the cellular sample 103. The stimulating signal 105 can be configured to cause a temporary change in the electrical properties of the cells within the cellular sample 103. The change can be temporary so that once the stimulating signal 105 is no longer applied to the cellular sample 103 the electrical properties of the cellular sample 103 return to their original state.

Fig. 3 shows a single cell 301 within the cellular sample 103. It is to be appreciated that the cellular sample 103 would comprise a plurality of cells 301 and can comprise different types of cells. The cell 301 comprises a nucleus 303, intracellular medium 305 and a plasma membrane 307. The nucleus 303 and the intracellular medium 305 are provided within the plasma membrane 307. Extra cellular medium 309 is provided outside of the plasma membrane 307.

Fig. 3 also shows a representation 311 of the electrical properties of the cell 301. Each of the components of the cell provides an impedance to an electrical signal. These impedances can be modified by the stimulating signals 105 applied to the cells 301. The representation 311 of the cell 301 shown in Fig. 3 shows the impedances of the components of the cells. However, it is to be appreciated that other electrical properties of the cell 301 could also be modified by the stimulating signals 105.

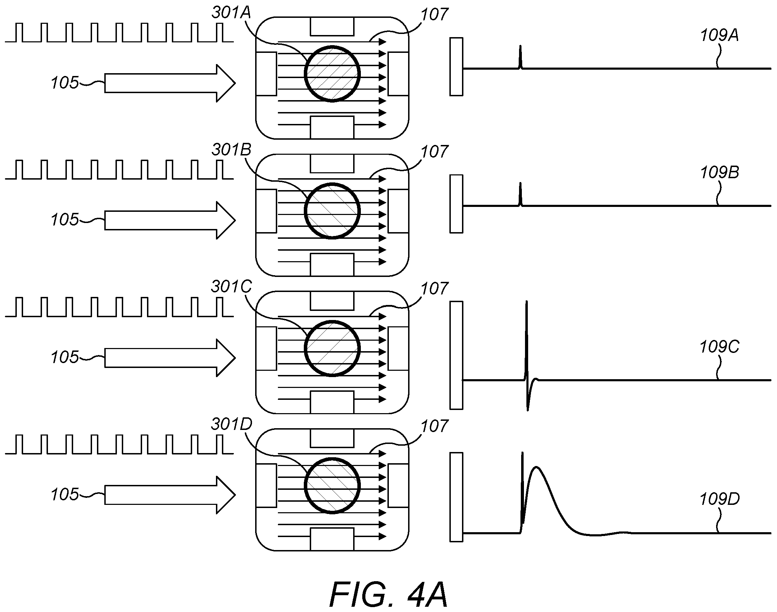

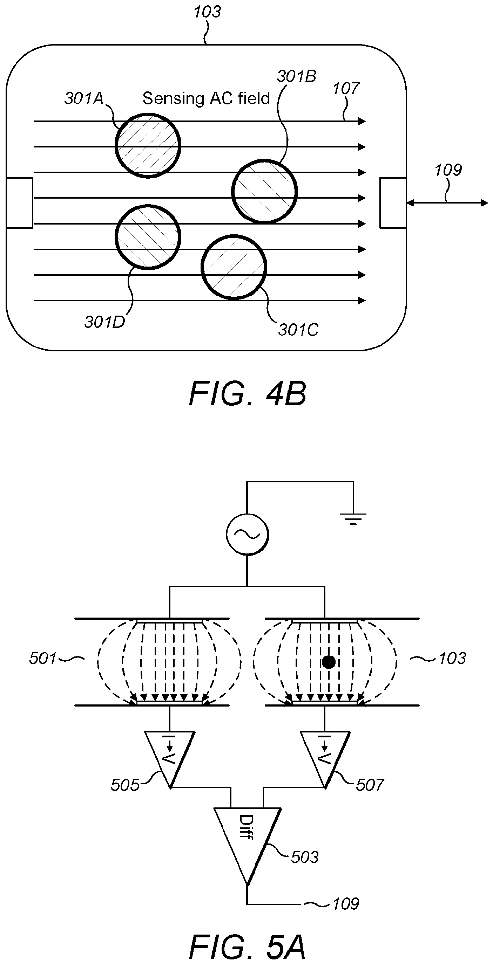

Figs. 4A and 4B schematically show how a database of electrical output signal values can be created for use during spectroscopic analysis of the electrical output signals 109 from a cellular sample 103.

The database can be created by applying a stimulating signal 105 and an electrical input signal 107 at a predetermined frequency to a known type of cell. The response in the electrical output signal 109 from the known type of cell for the stimulating signal 105 and the predetermined frequency of electrical input signal 107 can then be recorded in a database or other suitable register.

Fig. 4A shows a stimulating signal 105 and an electrical input signal 107 being applied to four different types of cell 301A to 301D. In order to enable the database to be created the known samples of cells 301A to 301D can be in-vitro samples.

In this example the stimulating signal 105 comprises an electrical pulse train having a defined frequency and amplitude. The same stimulating signal 105 is applied to each of the different types of cell 301A to 301D. The stimulating signal 105 will cause a different modification of the electrical properties for each of the different types of cell 301A to 301D.

An electrical input signal 107 having a single predetermined frequency is applied to each of the different types of cell 301A to 301D and the electrical output signal 109 for these different type for cell 301A to 301D is detected. The same electrical input signal 107 having the same frequency is applied to each of the different types of cell 301A to 301D so as to enable the different responses to be compared.

The different electrical output signals 109A to 109D provided by the cells 301A to 301D can be detected by any suitable means. As shown in Fig. 4A each of the different types of cell 301A to 301D provides a different response in the electrical output signal 109A to 109D. The different responses can be recorded and registered in a database.

In the example shown in Fig. 4A a single frequency is used for the electrical input signal 107 to provide the output signal. This provides an indication of the response of the cells 301A to 301D for a given frequency of electrical input signal 107 and for a given stimulation signal 105. In order to create a database, the process shown in Fig. 4A would be repeated with other electrical input signals at different frequencies and waveforms. This enables the output signals for different electrical input signal 107 to be recorded in the database. This enables the database to provide a record of the output signals 109 expected for different types of cells 301A to 301D for different input frequencies.

Fig. 4B shows a cellular sample 103 comprising the different types of cells being analysed. The cellular sample 103 can be an in-vivo sample comprising unknown types of cells.

A stimulating signal 105 is provided to the cellular signal to provide a modified electrical property of the cellular sample 103. The stimulating signal 105 is the same type of signal that is used to create the data in the database. An electrical input signal 107 can be provided simultaneously to the stimulating signal 105. The electrical input signal 107 comprises at least one frequency and waveform that was used as an input signal to create the data in the database.

The input electrical signal 107 passes through the cellular sample 103 to provide an output signal 109 and so the output signal 109 is determined by the electrical properties of the cells 301A to 301 D that are modified by the stimulating signal 105. The response provided in the output signal 109 can be correlated with the output signals in the database to identify the types of cells that are in the cellular sample 103.

In the example of Fig. 4B there are different types of cells 301A to 301D in the cellular sample 103 and so the output signals 109 provided by this cellular sample 103 would show the responses shown in Fig. 4A. By using a plurality of different electrical input signals at a plurality of different frequencies and waveforms, the response of the cellular sample 103 can be analysed to determine the types of cell within the cellular sample 103.

In the above example the same stimulating signal 105 is used for the obtaining the different output signals 109. The different stimulating signals 105 could have different properties such as different frequencies or could be different types of stimulating signals 105.

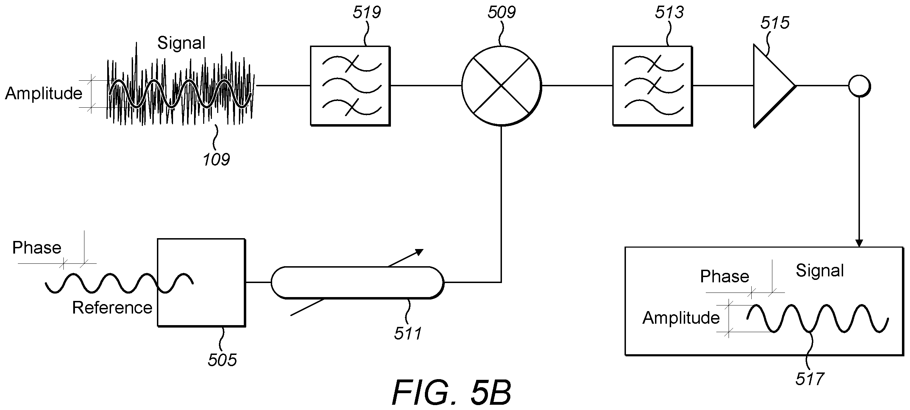

Fig. 5A and 5B schematically show how the cell sample signals can be isolated and obtained from in-vitro measurements.

Fig. 5A shows an example that can be used to isolate electrical signals from a cellular sample 103. In the example of Fig. 5A a common-mode background subtraction technique is used to isolate electrical signals from individual cellular sample 103 under in-vitro measurement. The common-mode background subtraction technique can reduce the noise that is present in the isolated electrical signals. The electrical signals that are obtained from components of the cells within the cellular sample 103 can be stored in one or more databases and used for comparative analysis in both in-vitro and in-vivo analysis of unknown cellular samples 103.

In Fig. 5A a background reference sample 501 comprising a cellular sample within a medium or incubating solution is positioned between two electrodes so that a background reference electrical signal 505 can be obtained from the background reference sample 501. The reference sample 501 can be an in-vitro sample. The background reference sample 501 receives the same electrical input signal 107 and stimulation signal 105 as the cellular sample 103.

The cellular sample 103 of unknown cell type is also positioned between two electrodes. The cellular sample 103 can be provided within the same medium or incubating solution as that of the background reference sample 501. An electrical signal 507 can be obtained from the cellular sample 103 and the background reference output signal 505 can be electronically subtracted from output signal 507 of the cellular sample 103 by a differential circuit operator 503. The subtraction eliminates any shared or common-mode background noises. The resultant electrical output signal 109 can be provided as an input for a lock-in amplifier as shown in Fig. 5B.

In the example shown in Fig. 5A, the background reference signal 505 and electrical signal 507 from the in-vitro cellular sample 103 are obtained at the same time. In other examples the background reference signal could be obtained from prior experiments and could be retrieved from a database or other register when needed.

In the example shown in Fig. 5A the cellular sample 103 is an in-vitro sample. The cellular sample can be stimulated using the same stimulating signal 105 used for the reference sample 501. The same frequency of electrical input signal 107 that is used for the reference sample 501 is also used for the cellular sample 103 that is being analysed.

In other examples the cellular sample 103 that is being analysed could be an in-vivo sample. In such examples the arrangement shown in Fig. 5A would not be used and the output from the in-vivo sample could be provided as an input for a lock-in amplifier as shown in Fig. 5B without first being subtracted from a reference signal.

Fig. 5B shows an example of a phase lock mechanism that can be used to improve the accuracy with which the phase-locked output signals 517 detected from an in-vivo or in-vitro cellular sample 103 can be correlated with one or more electrical signals from the database.

The electrical input signal 107 from apparatus 101 also serves as an electrical reference signal for the phase lock mechanism in the lock-in amplifier shown in Figure 5B.

The output signal 109 from the differential circuit operator 503 and the reference signal 107 can then be mixed using a phase lock mechanism comprising various modules as shown in Fig. 5B.

The phase lock mechanism is configured to find a signal corresponding to the reference signal 107 generated by a reference signal detector 505 in the output signal 109 from the cellular sample 103. The phase lock mechanism comprises a mixer 509 that is configured to mix the reference signal 505 with the output signal 109.

The phase lock mechanism comprises a first filter 519 that is configured to filter the output signal 109 before it is mixed with the reference signal 505. The phase lock mechanism also comprises a phase shifter 511 configured to phase shift the reference signal 505 before it is provided to the mixer 509 and mixed with the output signal 109.

Where there is correlation between the reference signal 505 and the output signal 109, the output of the mixer 509 will be non-zero over a given time interval. The phase lock mechanism also comprises a low pass filter 513 that is configured to receive the mixed signal from the mixer 509 and identify the part of output signal 109 that corresponds with the reference signal 505 by averaging the output of the mixer 509. The filtered output is provided to an amplifier 515 which then provides the phase locked output signal 517.

Fig. 6 is a plot that shows the frequency response of different parts of a cell 301. This plot demonstrates that different parts of cell 301 have different frequency responses. The example plot shown in Fig. 6 shows the characteristic zones within an impedance curve where the permittivity of different parts of the cell 301 are depended upon the frequency of an applied electric field.

The characteristic zones of respective parts of a cell are indicated by the dashed lines in Fig. 6. In the example shown in Fig. 6 the characteristic zone of the cell size is between 10⁴ Hz and 10⁵ Hz. The characteristic zone of the membrane is centred around 10⁶ Hz. The characteristic zone of the cytoplasm is centred around 10⁷ Hz and the characteristic zone of the vacuoles extends up to 10⁸ Hz. The stimulating signal 105 causes a change in the electrical output signal 109 for each given characteristic zone, since each part of the cell is affected by the stimulating signal 105. This in turn allows differentiation between the same component of different cell types, since this component might respond differently to the stimulation for each cell type. Therefore by providing an electrical input signal 107at different frequencies the different parts of the cell 301 can be interrogated.

This technique can be used to analyse different parts of the cells within the cellular sample 103. This can enable different parts of the cells to be interrogated and matched to reference signals to enable the type of cells within the cellular sample 103 to be identified.

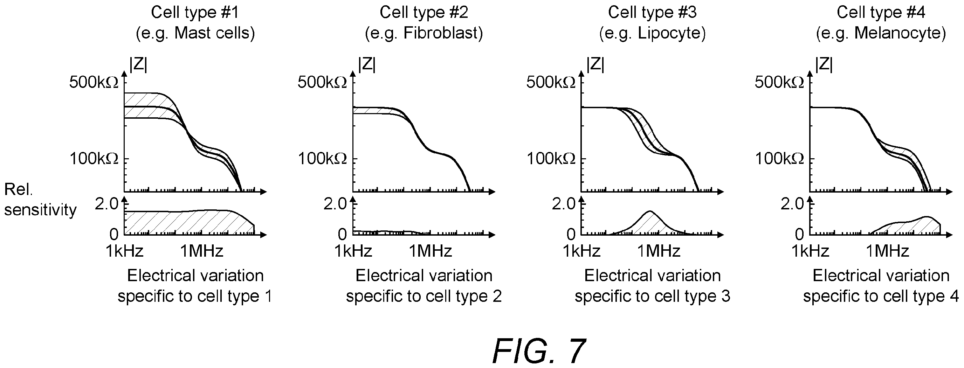

Fig. 7 shows the unique impedance response curves for four different types of cell 301. The first type of cell comprises mast cells, the second type of cell comprises fibroblast cells, the third type of cell comprises lipocyte cells and the fourth type of cells comprises melanocyte cells.

In order to obtain the data shown in the impedance response curves samples comprising the types of cells were stimulated using the same type of stimulating signal 105 and the impedance was measured for a range of frequency of the input signals 107. In this example the stimulating signal 105 comprises an electrical pulse train.

The impedance response curves show a different dielectric variation for each of the different types of cells that can be detected using apparatus 101 and methods as described above. The different response can therefore be used to identify the different types of cells.

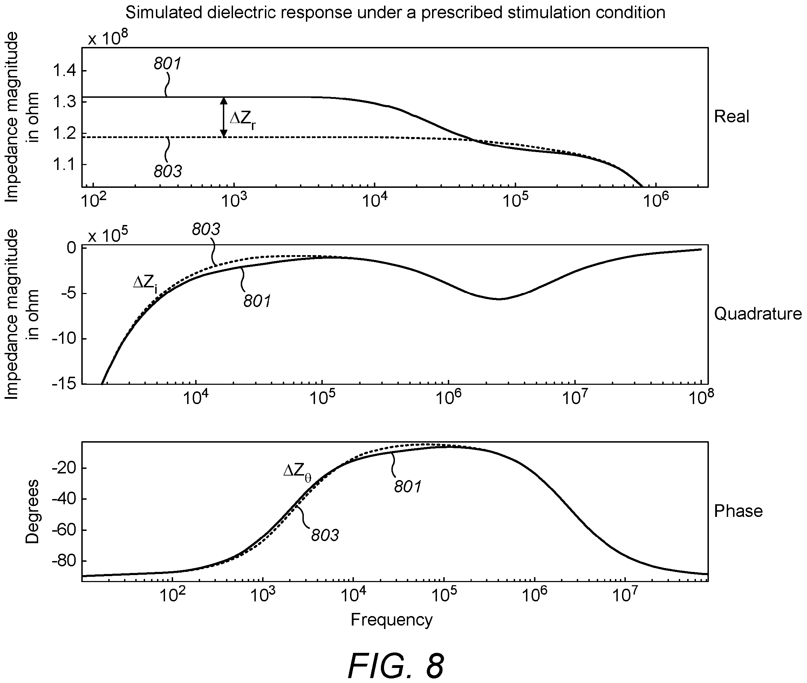

Fig. 8 shows an analytical simulation of expected magnitude responses for a single cell when a stimulating signal 105 is applied. This simulation shows the existence of a cell's unique dielectric modifications in response to a stimulating signal 105. In this simulation the stimulating signal 105 comprises an electrical pulse train. In this example the stimulating signal 105 causes a change in the dielectric properties of the cells.

The first curves 801 show the unstimulated responses which are obtained by providing an input signal 107 without providing a stimulating signal 105. The second curves 803 show the stimulated responses which are obtained by providing an input signal 107 while a stimulating signal 105 is also provided to the cellular sample 103. The differences in the stimulated and unstimulated responses show changes caused by the stimulating signal 105 provide unique identification characteristics for a particular type of cell. The stimulating signals 105 can be selected to provide larger modifications in the electrical properties of a given cell type or cellular component of a given cell type as these can provide for more accurate identification of the types of cell.

In Fig. 8 the real, quadrature and phase responses are shown in three separate plots to show the frequency ranges in which the stimulating signal causes a modification of the electrical properties of the cell.

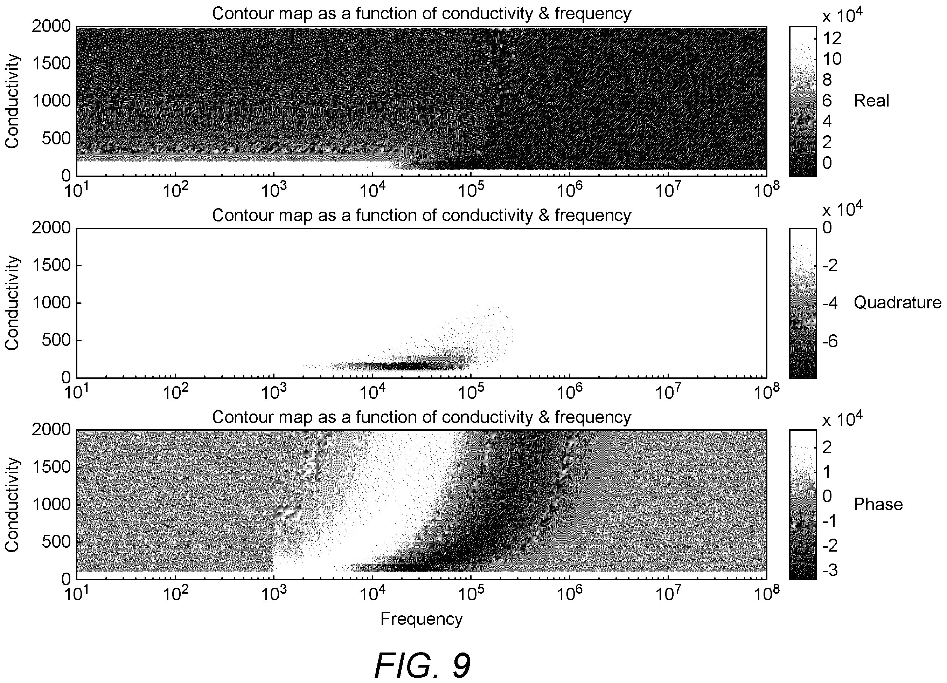

Fig. 9 shows an analytical simulation of expected magnitude responses and profile for a single cell when a stimulating signal 105 is applied under different electrical conductivities of extracellular fluid. In this example the stimulating signal 105 comprises an electrical pulse train.

The plots shown in Fig. 9 show that the modifications of the electrical properties for a given stimulating signal 105 are predictable for cells found within different environments. These different environments could be different organs or tissues, which would differ in the composition of the extracellular fluid and thus in the electrical conductivity of the extracellular fluid.

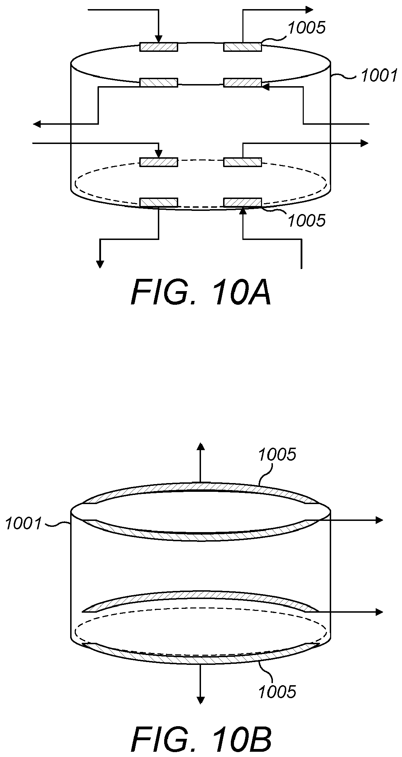

Figs. 10A to 10C show practical implementations and set ups that can be used in examples of the disclosure.

Fig. 10A shows a first experimental system that can be used to provide stimulating signals 105 such as electrical signals as well as to provide electrical input signals 107 and to receive electrical output signals 109.

The system comprises a microfluidic chamber 1001. The cellular sample 103 can be provided within the microfluidic chamber 1001. A plurality of electrodes 1005 are coupled to the microfluidic chamber 1001. The electrodes 1005 are coupled to the microfluidic chamber 1001 so as to enable electrical signals to be provided from the electrodes 1005 to the cellular sample 103. In the example of Fig. 10A the electrodes 1005 comprise small conductive pads that enable a localised signal to be provided to the cellular sample 103.

In the example shown in Fig. 10A four sets of electrodes 1005 are provided on the microfluidic chamber 1001. This can enable different stimulating signals 105 to be provided at the same time or can enable output signal 109 to be obtained from different parts of the cellular sample 103.

Fig. 10B shows another experimental system with a different arrangement of electrodes 1005. In this example a first set of electrodes 1005 is provided on an upper surface of the microfluidic chamber 1001 and a second set of electrodes 1005 is provided on the lower surface of the microfluidic chamber 1001. One of the sets of electrodes 1005 can be used to provide the stimulating signal 105 while the other set of electrodes 1005 can be used to provide the electrical input signal 107 and obtain the output signal 109.

In the example of Fig. 10B the electrodes have a semi-circular shape that extends along a surface of the microfluidic chamber 1001. This provides for non-localised signals.

It is to be appreciated that other arrangements of the electrodes 1005 could be used in other examples of the disclosure.

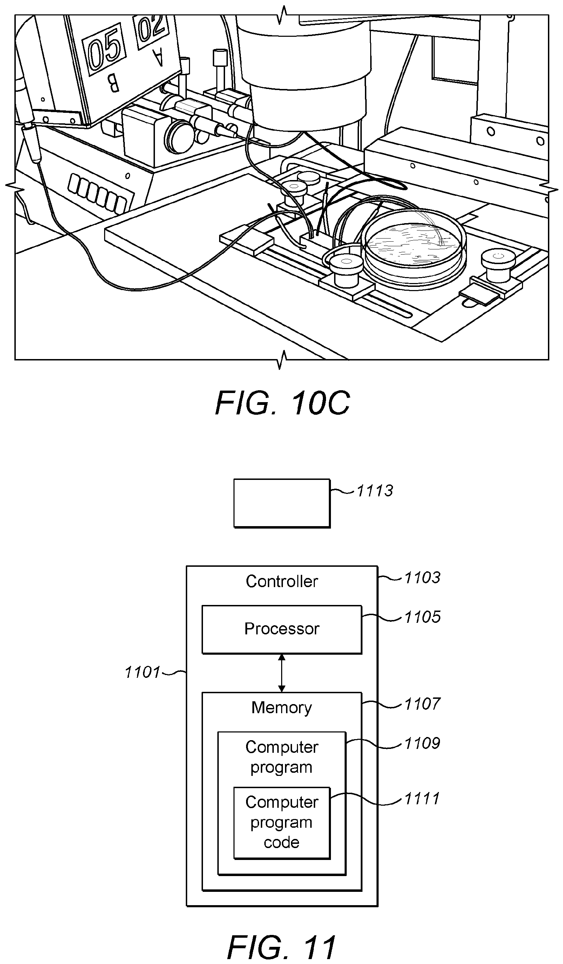

Fig. 10C is a photograph of an experimental set up that has been used to test examples of the disclosure. The experimental set up comprises a microfluidic chamber 1001 with 4-wire configuration. Micro-patterned planar wires are embedded within the microfluidic chamber 1001 however these are not visible in Fig. 10C. The exposed wires connect with these micro-patterned planar wires to link the microfluidic chamber 1001 with external apparatus for providing and detecting respective signals. A biological fluidic medium is configured to supply the microfluidic chamber 1001 with fresh, physiologically relevant fluids with varying conductivities to simulate the real tissue environment.

Fig. 11 schematically illustrates a controller apparatus 1101 according to examples of the disclosure. The controller apparatus 1101 illustrated in Fig. 11 can be a chip or a chip-set. In some examples the controller apparatus 1101 can be provided within a computer or other devices that be configured to provide and receive signals.

In the example of Fig. 11 the controller apparatus 1101 comprises a controller 1103. In the example of Fig. 11 the implementation of the controller 1103 can be as controller circuitry. In some examples the controller 1103 can be implemented in hardware alone, have certain aspects in software including firmware alone or can be a combination of hardware and software (including firmware).

As illustrated in Fig. 11 the controller 1103 can be implemented using instructions that enable hardware functionality, for example, by using executable instructions of a computer program 1109 in a general-purpose or special-purpose processor 1105 that can be stored on a computer readable storage medium (disk, memory etc.) to be executed by such a processor 1105.

The processor 1105 is configured to read from and write to the memory 1107. The processor 1105 can also comprise an output interface via which data and/or commands are output by the processor 1105 and an input interface via which data and/or commands are input to the processor 1105.

The memory 1107 is configured to store a computer program 1109 comprising computer program instructions (computer program code 1111) that controls the operation of the controller apparatus 1101 when loaded into the processor 1105. The computer program instructions, of the computer program 1109, provide the logic and routines that enables the controller apparatus 1101 to perform the methods illustrated in Fig. 2 The processor 1105 by reading the memory 1107 is able to load and execute the computer program 1109.

The controller apparatus 1101 therefore comprises: at least one processor 1105; and at least one memory 1107 including computer program code 1111, the at least one memory 1107 and the computer program code 1111 configured to, with the at least one processor 1105, cause the controller apparatus 1101 at least to perform: providing 201 a stimulating signal 105 to a cellular sample 103 wherein the stimulating signal 105 stimulates the cellular sample 103 so as to modify one or more electrical properties of the cellular sample 103; providing 203 a plurality of electrical input signals 107 having a plurality of different frequencies to the cellular sample 103; receiving 205 electrical output signals 109 from the cellular sample 103 where the electrical output signals 109 correspond to the electrical input signals 107 and the values of the electrical output signals 109 are determined by the modified electrical properties of the cellular sample 103; and enabling 207 spectroscopic analysis of the electrical output signals 109 to identify a type of cell in the cellular sample 103 from the modified electrical properties.

As illustrated in Fig. 11 the computer program 1109 can arrive at the controller apparatus 1101 via any suitable delivery mechanism 1113. The delivery mechanism 1113 can be, for example, a machine readable medium, a computer-readable medium, a non-transitory computer-readable storage medium, a computer program product, a memory device, a record medium such as a Compact Disc Read-Only Memory (CD-ROM) or a Digital Versatile Disc (DVD) or a solid state memory, an article of manufacture that comprises or tangibly embodies the computer program 1109. The delivery mechanism can be a signal configured to reliably transfer the computer program 1109. The controller apparatus 1101 can propagate or transmit the computer program 1109 as a computer data signal. In some examples the computer program 109 can be transmitted to the controller apparatus 1101 using a wireless protocol such as Bluetooth, Bluetooth Low Energy, Bluetooth Smart, 6LoWPan (IPᵥ6 over low power personal area networks) ZigBee, ANT+, near field communication (NFC), Radio frequency identification, wireless local area network (wireless LAN) or any other suitable protocol.

The computer program 1109 comprises computer program instructions for causing a controller apparatus 1101 to perform at least the following: providing 201 a stimulating signal 105 to a cellular sample 103 wherein the stimulating signal 105 stimulates the cellular sample 103 so as to modify one or more electrical properties of the cellular sample 103; providing 203 a plurality of electrical input signals 107 having a plurality of different frequencies to the cellular sample 103; receiving 205 electrical output signals 109 from the cellular sample 103 where the electrical output signals 109 correspond to the electrical input signals 107 and the values of the electrical output signals 109 are determined by the modified electrical properties of the cellular sample 103; and enabling 207 spectroscopic analysis of the electrical output signals 109 to identify a type of cell in the cellular sample 103 from the modified electrical properties.

The computer program instructions can be comprised in a computer program 1109, a non-transitory computer readable medium, a computer program product, a machine readable medium. In some but not necessarily all examples, the computer program instructions can be distributed over more than one computer program 1109.

Although the memory 1107 is illustrated as a single component/circuitry it can be implemented as one or more separate components/circuitry some or all of which can be integrated/removable and/or can provide permanent/semi-permanent/ dynamic/cached storage.

Although the processor 1105 is illustrated as a single component/circuitry it can be implemented as one or more separate components/circuitry some or all of which can be integrated/removable. The processor 1105 can be a single core or multi-core processor.

References to "computer-readable storage medium", "computer program product", "tangibly embodied computer program" etc. or a "controller", "computer", "processor" etc. should be understood to encompass not only computers having different architectures such as single /multi- processor architectures and sequential (Von Neumann)/parallel architectures but also specialized circuits such as field-programmable gate arrays (FPGA), application specific circuits (ASIC), signal processing devices and other processing circuitry. References to computer program, instructions, code etc. should be understood to encompass software for a programmable processor or firmware such as, for example, the programmable content of a hardware device whether instructions for a processor, or configuration settings for a fixed-function device, gate array or programmable logic device etc.

As used in this application, the term "circuitry" can refer to one or more or all of the following:
(a) hardware-only circuitry implementations (such as implementations in only analog and/or digital circuitry) and
(b) combinations of hardware circuits and software, such as (as applicable):
   (i) a combination of analog and/or digital hardware circuit(s) with software/firmware and
   (ii) any portions of hardware processor(s) with software (including digital signal processor(s)), software, and memory(ies) that work together to cause an apparatus, such as a mobile phone or server, to perform various functions and
(c) hardware circuit(s) and or processor(s), such as a microprocessor(s) or a portion of a microprocessor(s), that requires software (e.g. firmware) for operation, but the software can not be present when it is not needed for operation.
This definition of circuitry applies to all uses of this term in this application, including in any claims. As a further example, as used in this application, the term circuitry also covers an implementation of merely a hardware circuit or processor and its (or their) accompanying software and/or firmware. The term circuitry also covers, for example and if applicable to the particular claim element, a baseband integrated circuit for a mobile device or a similar integrated circuit in a server, a cellular network device, or other computing or network device.

The blocks illustrated in Fig. 2 can represent steps in a method and/or sections of code in the computer program 1109. The illustration of a particular order to the blocks does not necessarily imply that there is a required or preferred order for the blocks and the order and arrangement of the blocks can be varied. Furthermore, it can be possible for some blocks to be omitted.

The systems, apparatus 101, methods and computer programs 1109 can use machine learning which can include statistical learning. Machine learning is a field of computer science that gives computers the ability to learn without being explicitly programmed. The computer learns from experience E with respect to some class of tasks T and performance measure P if its performance at tasks in T, as measured by P, improves with experience E. The computer can often learn from prior training data to make predictions on future data. Machine learning includes wholly or partially supervised learning and wholly or partially unsupervised learning. It may enable discrete outputs (for example classification, clustering) and continuous outputs (for example regression). Machine learning may for example be implemented using different approaches such as cost function minimization, artificial neural networks, support vector machines and Bayesian networks for example. Cost function minimization may, for example, be used in linear and polynomial regression and K-means clustering. Artificial neural networks, for example with one or more hidden layers, model complex relationship between input vectors and output vectors. Support vector machines can be used for supervised learning. A Bayesian network is a directed acyclic graph that represents the conditional independence of a number of random variables.

Examples of the disclosure therefore provide for apparatus, methods and computer programs for detecting types of cells within a cellular sample 103. The use of a stimulating signal 105 to trigger a response provides for a measurable modification in the electrical properties of the cell which enables the identifying characteristics of the cells to be accurately identified and compared with known reference signals.

The system can also be used on in-vivo cellular samples which provides for a non-invasive system for recognizing cells. This can enable the system to be used on a living subject and can avoid the need for invasive procedures such as biopsies.

The use of the phase locked mechanism can enable the output signals to be tuned to a very specific frequency. This can enable the output signals 109 to be correlated with the reference signals even if there is low signal to noise ratio in the output signals 109.

The term 'comprise' is used in this document with an inclusive not an exclusive meaning. That is any reference to X comprising Y indicates that X may comprise only one Y or may comprise more than one Y. If it is intended to use 'comprise' with an exclusive meaning then it will be made clear in the context by referring to "comprising only one..." or by using "consisting".

In this description, reference has been made to various examples. The description of features or functions in relation to an example indicates that those features or functions are present in that example. The use of the term 'example' or 'for example' or 'can' or 'may' in the text denotes, whether explicitly stated or not, that such features or functions are present in at least the described example, whether described as an example or not, and that they can be, but are not necessarily, present in some of or all other examples. Thus 'example', 'for example', 'can' or 'may' refers to a particular instance in a class of examples. A property of the instance can be a property of only that instance or a property of the class or a property of a sub-class of the class that includes some but not all of the instances in the class. It is therefore implicitly disclosed that a feature described with reference to one example but not with reference to another example, can where possible be used in that other example as part of a working combination but does not necessarily have to be used in that other example.

Although examples have been described in the preceding paragraphs with reference to various examples, it should be appreciated that modifications to the examples given can be made without departing from the scope of the claims.

Features described in the preceding description may be used in combinations other than the combinations explicitly described above.

Although functions have been described with reference to certain features, those functions may be performable by other features whether described or not.

Although features have been described with reference to certain examples, those features may also be present in other examples whether described or not.

The term 'a' or 'the' is used in this document with an inclusive not an exclusive meaning. That is any reference to X comprising a/the Y indicates that X may comprise only one Y or may comprise more than one Y unless the context clearly indicates the contrary. If it is intended to use 'a' or 'the' with an exclusive meaning then it will be made clear in the context. In some circumstances the use of 'at least one' or 'one or more' may be used to emphasis an inclusive meaning but the absence of these terms should not be taken to infer any exclusive meaning.

The presence of a feature (or combination of features) in a claim is a reference to that feature or (combination of features) itself and also to features that achieve substantially the same technical effect (equivalent features). The equivalent features include, for example, features that are variants and achieve substantially the same result in substantially the same way. The equivalent features include, for example, features that perform substantially the same function, in substantially the same way to achieve substantially the same result.

In this description, reference has been made to various examples using adjectives or adjectival phrases to describe characteristics of the examples. Such a description of a characteristic in relation to an example indicates that the characteristic is present in some examples exactly as described and is present in other examples substantially as described.

Whilst endeavoring in the foregoing specification to draw attention to those features believed to be of importance it should be understood that the Applicant may seek protection via the claims in respect of any patentable feature or combination of features hereinbefore referred to and/or shown in the drawings whether or not emphasis has been placed thereon.

## Claims

1. An apparatus comprising means for:
providing a stimulating signal to a cellular sample wherein the stimulating signal stimulates the cellular sample so as to modify one or more electrical properties of the cellular sample;
providing a plurality of electrical input signals having a plurality of different frequencies to the cellular sample;
receiving electrical output signals from the cellular sample where the electrical output signals correspond to the electrical input signals and the values of the electrical output signals are based on the modified one or more electrical properties of the cellular sample; and
enabling spectroscopic analysis of the electrical output signals to identify a type of cell in the cellular sample from the modified one or more electrical properties.

2. An apparatus as claimed in claim 1 wherein the analysis comprises comparing the received output electrical signals with a database of known electrical output signal values of cellular samples so as to identify at least one cell type within the cellular sample.

3. An apparatus as claimed in 2 wherein the known electrical output signal values are obtained from in-vitro measurements of known cellular samples.

4. An apparatus as claimed in any preceding claim wherein the analysis comprises impedance spectroscopy of the electrical output signals.

5. An apparatus as claimed in any preceding claim wherein the plurality of electrical input signals comprise a carrier waveform where the carrier waveform is frequency locked to a preselected frequency.

6. An apparatus as claimed in claim 5 where the preselected frequency corresponds to a component of the cellular sample.

7. An apparatus as claimed in any preceding claim wherein the plurality of electrical input signals comprise a phase locked signal.

8. An apparatus as claimed in any preceding claim wherein the means are for providing a plurality of different stimulating signals at different times to enable a plurality of different modified one or more electrical properties of the cellular sample to be analysed using the spectroscopic analysis.

9. An apparatus as claimed in any preceding claim wherein the one or more electrical properties that are modified by the stimulating signal comprise one or more of resistance, capacitance, impedance, dielectrics, phase.

10. An apparatus as claimed in any preceding claim wherein the stimulating signal comprises one or more of electrical signals, mechanical signals, acoustic signals, photonic signals, magnetic signals, electromagnetic signals.

11. An apparatus as claimed in any preceding claim wherein at least one of the stimulating signal or the plurality of electrical input signals is provided in programmed waveforms.

12. An apparatus as claimed in any preceding claim wherein the stimulating signal causes a temporary modification of the one or more electrical properties of the cellular sample.

13. An apparatus as claimed in any preceding claim wherein the cellular sample comprises an in-vivo sample.

14. A method comprising:
providing a stimulating signal to a cellular sample wherein the stimulating signal stimulates the cellular sample so as to modify one or more electrical properties of the cellular sample;
providing a plurality of electrical input signals having a plurality of different frequencies to the cellular sample;
receiving electrical output signals from the cellular sample where the electrical output signals correspond to the electrical input signals and the values of the electrical output signals are based on the modified one or more electrical properties of the cellular sample; and
enabling spectroscopic analysis of the electrical output signals to identify a type of cell in the cellular sample from the modified one or more electrical properties.

15. A computer program comprising computer program instructions that, when executed by processing circuitry, cause:
providing a stimulating signal to a cellular sample wherein the stimulating signal stimulates the cellular sample so as to modify one or more electrical properties of the cellular sample;
providing a plurality of electrical input signals having a plurality of different frequencies to the cellular sample;
receiving electrical output signals from the cellular sample where the electrical output signals correspond to the electrical input signals and the values of the electrical output signals are based on the modified one or more electrical properties of the cellular sample; and
enabling spectroscopic analysis of the electrical output signals to identify a type of cell in the cellular sample from the modified one or more electrical properties.
